(19)

Europäisches
Patentamt

European
Patent Office

Office européen
des brevets

(11) **EP 4 786 045 A2**

(12) **EUROPEAN PATENT APPLICATION**

(43) Date of publication:
**05.08.2026   Bulletin 2026/32**

(21) Application number: **26155461.2**

(22) Date of filing: **30.01.2026**

(51) International Patent Classification (IPC):
***B01J 23/26*** (2006.01)

(52) Cooperative Patent Classification (CPC):
**B01J 23/26; B01J 37/0207; C07C 5/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **30.01.2025   IN 202521007857**

(71) Applicant: **Indian Oil Corporation Limited
Mumbai 400 051 (IN)**

(72) Inventors:
- **MANJUNATHAN, Pandian
121007 Faridabad (IN)**
- **CHIDAMBARAM, Velusamy
121007 Faridabad (IN)**
- **DOOSA, Hima Bindu
121007 Faridabad (IN)**
- **KARTHIKEYANI, Arumugam Velayutham
121007 Faridabad (IN)**
- **PULIKOTTIL, Alex Cheru
121007 Faridabad (IN)**

(74) Representative: **Pons IP
Glorieta Rubén Darío 4
28010 Madrid (ES)**

(54) **NANOCRYSTALLINE COMPOSITE CATALYST FOR ALKANE DEHYDROGENATION AND PROCESS FOR PREPARATION THEREOF**

(57)    The present invention discloses a composite catalyst for non-oxidative dehydrogenation of lighter alkanes, comprising: a) a catalyst support in an amount of 70-90wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst; b) a first transition metal oxide in an amount of 5-25 wt.% of the composite catalyst; and c) one or more second transition metal oxide in an amount of 1-10 wt.% of the composite catalyst. The present invention also discloses a process for preparation of the composite catalyst for non-oxidative dehydrogenation of lighter alkanes.

EP 4 786 045 A2

**Description**

## FIELD OF THE INVENTION

The present invention discloses a composite catalyst for non-oxidative dehydrogenation of lighter alkanes, comprising: a) a catalyst support in an amount of 70-90 wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst; b) a first transition metal oxide in an amount of 5-25 wt.% of the composite catalyst; and c) one or more second transition metal oxide in an amount of 1-10 wt.% of the composite catalyst. The identified catalyst composition possesses a unique combination of activity, selectivity and chromium stability after substitution of chromium with another transition metals. The composite catalyst preparation methodology of the present invention enabled to improve the dehydrogenation behaviour of propane and propylene selectivity at high propane concentration. The present invention provides the dehydrogenation composite catalyst exhibiting better activity at lower amount of chromium oxide (8-12 wt.%) against the commercial catalyst composition containing chromium oxide as active component.

## BACKGROUND OF THE INVENTION

Light olefins (C2 to $C_4^=$) such as ethylene, propylene, butylene and isobutylene are the most important and the key fundamental building blocks in petrochemical industries find various applications in the polymers, chemicals, oil-and-gas, and other branches of industry. Currently, light olefins are commercially produced through the process namely steam cracking, fluid catalytic cracking (FCC) and methanol-to-olefins (MTO). Alternatively, the availability of large reserves of shale gas opened the window of opportunity to overcome the rapidly growing demand for light olefins by dehydrogenation of light alkanes, which is an attractive and low energy consumption process.

The dehydrogenation of alkane involves the cleavage of two C-H bonds of alkane with the formation of a hydrogen molecule. Various solid catalytic materials with the metal/ metal oxide supported systems namely supported $CrO_x$, $VO_x$, Pt-Sn, Ni-Sn, $GaO_x$, Fe-Zn, $MoO_3$, ZnO; and metal incorporated mesoporous materials have been exploited for light alkane dehydrogenation reactions. However, the challenges continue in developing catalyst to produce olefins with high selectivity and improve catalyst stability from deactivation. Therefore, a lot of efforts have been made to overcome the issue by improving the catalyst properties. The following points are most important in catalyst development to achieve high olefin selectivity and catalyst stability, as follows: (a) suppressing the side reactions namely cracking of reactant alkanes and dimerization of produced alkenes by acidic sites of catalyst in reaction environment, (b) restricting/ minimizing the oligomerization of alkenes to avoid coke formation, (c) utilization of stabilized catalyst composition to avoid sintering of active component responsible for activity.

The few technologies on propane dehydrogenation reactions are commercialized and employ C3, C4 and C5 as feeds and the processes are CATOFIN, STAR and OLEFLEX. The CATOFIN process employs the catalyst composition namely alumina supported with 18-20 wt.% $CrO_x$ (~23 - 26wt.% $Cr_2O_3$) and 1-2 wt.% $Na_2O/K_2O$ as promoter. The catalyst system of OLEFLEX process comprised of alumina supported with 1 wt.% Pt, 1-2 wt% Sn, 0-1 wt% Na or K, whereas the STAR process employs 0.01-5 wt.% Pt, 0.1-5 wt% Sn supported on zinc aluminate (Ca-Mg aluminate binder). The DOW Fcdh employs supported Pt-Ga as a catalyst, wherein the catalyst support is alumina. Although the supported $CrO_x$- and Pt-based catalyst systems are commercially employed, the $CrO_x$-based catalyst has advantages over a Pt-based catalyst system owing to their low price and availability of raw material. However, $CrO_x$-based catalyst is easy to deactivate and requires frequent regeneration. Some of the prior-art documents are given below:

US 20040242945A1 inventions discloses catalyst containing metal aluminates of zinc, manganese, or calcium as catalyst support with or without platinum as active metal for dehydrogenation catalysts.

US20130165729A1 discloses platinum free catalyst comprising zinc and/or manganese aluminate, optionally further comprising of promoters namely either of sodium (Na), potassium (K), cesium (Cs), rubidium (Rb), strontium (Sr), barium (Ba), magnesium (Mg), calcium (Ca), gallium (Ga), germanium (Ge), tin (Sn), copper (Cu), Zirconium (Zr), cobalt (Co), tungsten (W) or mixtures thereof for dehydrogenation of C2-C8 alkanes particularly isobutane in a down flow fixed bed micro-catalytic reactor. The literature study has shown that the addition of zinc to platinum or chromium-containing catalysts exhibits increase in alkene selectivity during alkane dehydrogenation reactions.

US11040338B2 discloses an invention of supported platinum-tin (0.5%Pt - 1%Sn) based catalysts for dehydrogenation of light alkane preferably propane as a feed, wherein the support contains a combination of zeolite, particularly SAPO-34 (60%); metal aluminates namely zinc aluminate (28%) and calcium aluminate (12%); and a promoter that includes alkali metals namely lithium, rubidium, cesium and beryllium.

US4041099A discloses an invention of supported platinum-tin (Pt -Sn) based catalysts for dehydrogenation of light alkane dehydrogenation reaction.

CN111408370A discloses a supported PtZn (platinum-zinc) intermetallic alloy as a catalyst, wherein silica is used as a catalyst support, zinc is used as an auxiliary agent and platinum as an active component for propane dehydrogenation reaction under reduced environment using hydrogen gas.

US20100010280A1, US8063261B2 and US8101541B2 inventions disclose a stationary or fluid bed dehydrogenation catalyst containing alumina as a support, with chromium oxide (19wt%) using chromic acid as a precursor, which is particularly useful in vapor phase dehydrogenation.

US20050075243A1 discloses an invention for a stationary or fluid bed dehydrogenation catalyst containing alumina as a catalyst support, with oxides of chromium (19%), zirconium, magnesium and, preferably, an alkali metal added as promoters; and wherein chromic acid is used as a precursor for $Cr_2O_3$. The resultant catalyst demonstrates greater selectivity and olefin yield than prior art dehydrogenation catalysts containing aluminum and chromium only.

US20200147588A1 and US20210001316 provides a method for preparing a dehydrogenation catalyst containing alumina with different alumina phases may include gamma, eta, theta, alpha as support with chromium oxide (20wt%) as active sites and alkali metals namely sodium, lithium and potassium as promoters.

US20100312035A1 and US20030232720A1 discloses a catalyst composition containing alumina, lithium oxide, alkaline earth metal oxide, chromium oxide and sodium oxide; wherein chromium (VI) oxide ($CrO_3$) is used as precursor.

US8895468B2 and WO2014046659A1 relates to an invention for preparation of dehydrogenation catalyst employing silica-stabilized alumina as catalyst support, wherein the catalyst composition is comprised of $Cr_2O_3$, an alkali metal oxide, $SiO_2$ and $Al_2O_3$; the said catalyst is used to produce olefins from dehydrogenation of alkanes.

Advancement over prior art(s):

The rapidly growing demand for light olefins directly impacts on catalyst production and its revenue. As alkane dehydrogenation reaction is an endothermic process, high operation temperatures and harsh reaction conditions extensively limit the catalyst development. Pt and $CrO_x$-based catalysts have been applied in commercialized PDH processes on account of their excellent activity and propylene productivity. Furthermore, the development of dehydrogenation catalyst producing light olefins at high yield and the minimization of $CrO_x$ amount in catalyst composition makes the process more economically viable. This could be achieved by tailoring the structural and chemical properties of active $CrO_x$ species in catalyst composition, which could provide an excellent catalyst stability and activity.

Although $CrO_x$-based catalyst is employed for dehydrogenation of light alkanes, still it severely suffers from deactivation due to the coke formation by oligomerization of formed alkenes in the reaction environment. Consequently, frequent regeneration is desirable to reactivate the catalyst by burning of coke. The typical fixed-bed mediated dehydrogenation reaction process may suffer due to the time involved during regeneration and activation steps, which slows down the productivity of process. The dehydrogenation of alkanes under fluidization environment benefits in progressing the reaction by following the steps in sequence involving reaction - regeneration - activation, continuously. Besides, the heat generated during coke burning can be effectively utilized by supplying to reactor since dehydrogenation reaction is endothermic in nature. However, continuous regeneration at higher temperature may alter the properties of catalyst and catalyst support. Hence, the catalyst development with high thermal stability and retaining the activity is more desirable.

The present invention developed a process for preparation of a catalyst composition suitable for non-oxidative propane dehydrogenation reaction for providing high hydrothermal stability, high chromium stability, high resistant capacity against chromium leaching, high alkane conversion and higher olefin selectivity.

## OBJECTIVES OF THE INVENTION:

Primary objective of the present invention is to provide a catalyst or composite catalyst or catalyst composition suitable for non-oxidative dehydrogenation of lighter alkanes.

Another objective of the present invention is to provide a composite catalyst for non-oxidative dehydrogenation of lighter alkanes, comprising: a) a catalyst support in an amount of 70-90wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst; b) a first transition metal oxide in an amount of 5-25 wt.% of the composite catalyst; and c) one or more second transition metal oxide in an amount of 1-10 wt.% of the composite catalyst.

Another objective of the present invention is to provide a composite catalyst wherein the catalyst composition possesses a unique combination of activity, selectivity, and stability of chromium even after substitution of chromium with another transition metals.

Yet another objective of the present invention is to provide process for preparation of the composite catalyst to improve the dehydrogenation behaviour of lighter alkanes selectivity.

Yet one another objective of the present invention is to provide process for preparation of the composite catalyst to improve the dehydrogenation behaviour of propane and propylene selectivity at high propane concentration.

**SUMMARY OF THE INVENTION:**

The present invention discloses a composite catalyst for non-oxidative dehydrogenation of lighter alkanes, comprising: a) a catalyst support in an amount of 70-90wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst; b) a first transition metal oxide in an amount of 5-25 wt.% of the composite catalyst; and c) one or more second transition metal oxide in an amount of 1-10 wt.% of the composite catalyst.

In a feature of the present invention, the catalyst support is selected from alumina or aluminum oxide, aluminum monohydrate, aluminum trihydrate, gamma-alumina, eta-alumina, delta-alumina, silica-alumina and zeolites; wherein the rare-earth metals are selected from cerium, lanthanum, yttrium, scandium, samarium or mixtures thereof; and wherein the alkaline earth metals are selected from magnesium, calcium, strontium, rubidium, barium, or mixtures thereof.

In a feature of the present invention, the first transition metal oxide is in the range of 8-12 wt.% of the composite catalyst and is chromium oxide; and wherein the second transition metal oxide is in the range of 2-4 wt.% of the composite catalyst and is selected from iron oxide, cobalt oxide, nickel oxide, copper oxide, zinc oxide or mixtures thereof.

The present invention also discloses a process for preparing a composite catalyst for non-oxidative dehydrogenation of lighter alkanes, comprising the steps of: i) dissolving 42.1-63.2 grams of chromium nitrate nonahydrate and 7.3-14.7 grams of at least one second transition metal nitrate in deionized water to obtain a mixed metal nitrate solution; ii) impregnating the mixed metal nitrate solution by spraying on a calcined catalyst support-D to obtain an impregnated catalyst support-D; iii) drying the impregnated catalyst support-D at 100-150 °C overnight to obtain a dried catalyst, and iv) calcining the dried catalyst at a temperature in the range of 500 - 900 °C for 2 - 10 hours to obtain the composite catalyst having particle size about 20 to 150 $\mu$m.

In a feature of the present invention, the process used a fixed bed or fluidization reactor system.

In a feature of the present invention, the chromium nitrate nonahydrate corresponds to 8-12 wt.% $Cr_2O_3$ in the total composite catalyst; and wherein the second transition metal nitrates correspond to 2-4 wt.% of transition metal oxide (MO) in the total composite catalyst; wherein transition metal in the transition metal nitrate and in the transition metal oxide is selected from iron, cobalt, nickel, copper and zinc.

In a feature of the present invention, the impregnated catalyst support-D is dried at a temperature of 120 °C; and wherein the calcination is performed at a temperature of 650 °C for 2-10 hours.

In a feature of the present invention, the calcined catalyst support-D is prepared by a process comprising the steps of: i) mixing 500 - 600 grams of dispersible alumina, 500 - 600 grams of rare-earth (RE)-containing alumina precursor, 100 - 200 grams of magnesium-aluminum hydroxide or hydroxycarbonates, and 1800 - 2000 ml of deionized water to obtain a slurry; ii) transferring the slurry into an attritor and ball milling the slurry for 50 - 70 minutes to obtain a milled slurry; iii) adding 1000 - 1100 ml of a solution containing 40-60 wt.% of aluminum nitrate to the milled slurry with continuous stirring for 50-80 minutes to obtain a homogenized slurry; iv) spray drying the slurry followed by calcining at a temperature in the range of 500 - 900 °C for 4 - 10 hours to obtain the calcined catalyst support D.

**BRIEF DESCRIPTION OF THE ACCOMPANYING DRAWINGS/FIGURES:**

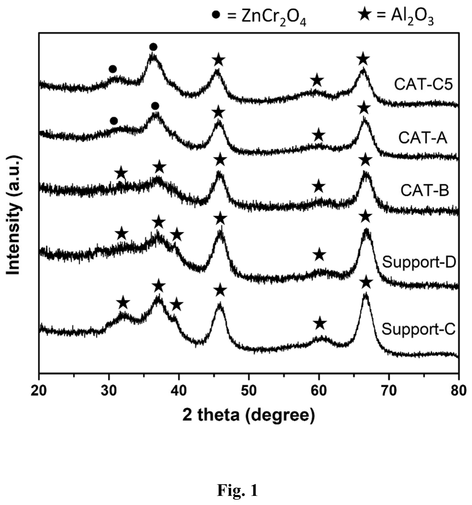

Fig. 1 illustrates XRD patterns of dehydrogenation catalysts and catalysts support. a) Support-C: $Mg-Al_2O_3$; b) Support-D: $RE-Mg-Al_2O_3$; c) CAT-B: $Cr_2O_3/RE-Mg-Al_2O_3$; d) CAT-A: $Cr_2O_3+ZnO/Mg-Al_2O_3$; e) CAT-C5: $Cr_2O_3-ZnO/RE-Mg-Al_2O_3$.

**DETAILED DESCRIPTION OF THE INVENTION:**

The present invention discloses a process of making multifunctional catalysts for non-oxidative dehydrogenation of light alkanes with catalyst containing lower amount of chromium oxide ($\leq$ 12% $Cr_2O_3$) in the total composition. The chromium oxide is transformed to mixed metal oxide by substitution using another transition metals like Fe, Co, Ni, Cu and Zn as structural modulator or chromium stabilizing agent. The catalyst preparation methodology employs a modified alumina support which is hydrothermally stable and resistant towards coke formation. In a typical experiment, the said catalyst exhibited ~46% propane conversion and 88% propylene selectivity.

The present invention relates to a catalyst composition for upgrading light alkanes namely $C_2$-$C_5$ hydrocarbons at high alkane concentration 30-90% balanced in nitrogen gas. More specifically, the present invention relates to the catalyst composition composed of mixed or composite metal oxide containing lower amount of chromium oxide (8-12wt.%) supported on rare-earth metal stabilized magnesium containing mesoporous microsphere alumina for better hydrothermal stability and excellent catalytic activity. The process for catalyst preparation involves the construction of mixed or composite metal oxide on catalyst support by employing chromium precursor along with another metal precursor for structural transformation. The neighbouring transition metal species in $MCr_2O_4$ could provide a better catalytic environment for reactant molecules against the $Cr_2O_3$ based catalyst. Importantly, the presence of alkaline-earth metal impacts on reducing the coke formation by decreasing the acidic sites.

The present invention discloses development of rare-earth metal stabilized magnesium containing mesoporous microsphere alumina support to improve the hydrothermal stability and ability to reduce the coke formation in dehydrogenation catalysts. Next, the invention deals with the preparation of nanocrystalline composite metal oxide catalysts by using less amount of chromium oxide ($\leq$12 wt.%) in combination with other transition metals as structural modulator namely oxides of iron (Fe), nickel (Ni), cobalt (Co), copper (Cu) and zinc (Zn). These transition metals act as chromium stabilizing agent to induce crystal structural transformation, which directly impacts on chemical properties of catalysts providing better availability of $Cr_2O_3$ sites, generation of defective sites and improves the chromium stability against the leaching. In addition, the introduction of alkaline earth metal (Mg) in alumina support by employing hydroxides or hydroxycarbonates of magnesium-aluminum benefits in reducing acidic property of support which thereby inhibit the coke formation and enhances the olefin selectivity.

In an aspect of the present invention, the present invention discloses a composite catalyst for non-oxidative dehydrogenation of lighter alkanes, comprising: a) a catalyst support in an amount of 70-90wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst; b) a first transition metal oxide in an amount of 5-25 wt.% of the composite catalyst; and c) one or more second transition metal oxide in an amount of 1-10 wt.% of the composite catalyst.

In an embodiment of the present invention, the catalyst support is selected from alumina or aluminum oxide, aluminum monohydrate, aluminum trihydrate, gamma-alumina, eta-alumina, delta-alumina, silica-alumina and zeolites.

In an embodiment of the present invention, the rare-earth metals are selected from cerium, lanthanum, yttrium, scandium, samarium or mixtures thereof.

In an embodiment of the present invention, the alkaline earth metals are selected from magnesium, calcium, strontium, rubidium, barium, or mixtures thereof.

In an embodiment of the present invention, the first transition metal oxide is in the range of 8-12 wt.% of the composite catalyst and is chromium oxide; and wherein the second transition metal oxide is in the range of 2-4 wt.% of the composite catalyst and is selected from iron oxide, cobalt oxide, nickel oxide, copper oxide, zinc oxide or mixtures thereof.

In another aspect of the present invention, the present invention discloses a process for preparing a composite catalyst for non-oxidative dehydrogenation of lighter alkanes, comprising the steps of: i) dissolving 42.1-63.2 grams of chromium nitrate nonahydrate and 7.3-14.7 grams of at least one second transition metal nitrate in deionized water to obtain a mixed metal nitrate solution; ii) impregnating the mixed metal nitrate solution by spraying on a calcined catalyst support-D to obtain an impregnated catalyst support-D; iii) drying the impregnated catalyst support-D at 100-150 °C overnight to obtain a dried catalyst, and iv) calcining the dried catalyst at a temperature in the range of 500 - 900 °C for 2 - 10 hours to obtain the composite catalyst having particle size about 20 to 150 $\mu$m.

In an embodiment of the present invention, the process used a fixed bed or fluidization reactor system.

In an embodiment of the present invention, the chromium nitrate nonahydrate corresponds to 8-12 wt.% $Cr_2O_3$ in the total composite catalyst; and wherein the second transition metal nitrates correspond to 2-4 wt.% of transition metal oxide (MO) in the total composite catalyst; wherein transition metal in the transition metal nitrate and in the transition metal oxide is selected from iron, cobalt, nickel, copper and zinc.

In an embodiment of the present invention, the impregnated catalyst support-D is dried at a temperature of 120 °C; and wherein the calcination is performed at a temperature of 650 °C for 2-10 hours.

In an embodiment of the present invention, the calcined catalyst support-D is prepared by a process comprising the steps of: i) mixing 500-600 grams of dispersible alumina, 500-600 grams of rare-earth (RE)-containing alumina precursor, 100-200 grams of magnesium-aluminum hydroxide or hydroxycarbonates, and 1800-2000 ml of deionized water to obtain a slurry; ii) transferring the slurry into an attritor and ball milling the slurry for 50-70 minutes to obtain a milled slurry; iii) adding 1000-1100 ml of a solution containing 40-60 wt.% of aluminum nitrate to the milled slurry with continuous stirring for 50-80 minutes to obtain a homogenized slurry; iv) spray drying the slurry followed by calcining at a temperature in the range of 500-900 °C for 4-10 hours to obtain the calcined catalyst support D.

In an embodiment of the present invention, the combination of $MCr_2O_4$ solid solution and $Cr_2O_3$ is present at a total chromium oxide concentration from about 8wt.% to about 12wt.% based on total composite catalyst weight.

In an embodiment of the present invention, the $MCr_2O_4$ is present at a total transition metal oxide concentration from about 2wt.% to about 4wt.% based on total composite catalyst weight.

In an embodiment of the present invention, the $MCr_2O_4$ active component (M = Fe, Co, Ni, Cu and Zn) consists of either $FeCr_2O_4$, $CoCr_2O_4$, $NiCr_2O_4$, $CuCr_2O_4$, $Zn Cr_2O_4$ or a combination of two or more in the composite catalyst.

In an embodiment of the present invention, the rare-earth stabilized alumina catalyst support contains cerium, lanthanum, yttrium, scandium, samarium or combination of two or more, at the concentration of 0.5 to 10wt.% of total catalyst weight.

In an embodiment of the present invention, the active metals are impregnated by co-impregnation of two or more metal nitrates or sulphates in a one step or two steps or three steps process by spraying the mixed metal salt solution on catalyst support.

In an embodiment of the present invention, the zinc nitrate is impregnated on catalyst support first and followed by impregnation of chromium salt in a one-step or two-step or three-step process by spraying the metal salt solution on support.

In an embodiment of the present invention, the metal-chelate or mixed metal chelate complex is used as a precursor to impregnate on catalyst support; wherein the zinc-chromium-chelate complex comprises of zinc-chromium oxide after calcination; the chelating agents may include citric acid, maleic acid, oxalic acid.

In an embodiment of the present invention, the rare-earth in alumina catalyst support substituted with metals selected from hafnium, zirconium, titanium, or combination of two or more, at the concentration of 0.5 to 10wt.% of total catalyst weight.

In an embodiment of the present invention, the lighter alkanes comprise $C_2$ to $C_5$, preferably $C_2$ to $C_4$.

The following are the advantages of present invention:

- The porosity of catalyst support is tailored by employing porous alumina precursor to achieve better chromium distribution to improve propane conversion and propylene selectivity.
- The hydrothermal stability of the dehydrogenation catalyst is improved by employing alumina support containing rare-earth metals like cerium, lanthanum, or other metals like zirconium, hafinum.
- The acidic properties responsible for coke formation via alkene oligomerization are suppressed by modulating the acidic sites in alumina support using alkaline-earth metals like magnesium. The support is formulated by employing magnesium precursor include hydroxide or hydroxycarbonates of magnesium-aluminum in combination with alumina precursor containing rare-earth metals like cerium, lanthanum, or other metal like zirconium, hafinum.
- The introduction of transition metals (M = Fe, Co, Ni, Cu and Zn) as structural modulators in $Cr_2O_3$ during catalyst preparation impacts on crystal structure due to the substitution of chromium with other transition metal. This results in structural transformation of $Cr_2O_3$ to $MCr_2O_4$ spinel/ mixed metal oxide/ solid solution (for example $Cr_2O_3$ to $MCr_2O_4$).
- Improved catalyst system with composition containing less chromium oxide content (≤12 wt.%) against the commercial catalyst containing 18-20 wt.% chromium oxide (1-2 wt.% $K_2O$ and 18-20 wt.% $CrO_x$ (i.e., ~ 23 -26 wt.% $Cr_2O_3$)).
- The formation of $MCr_2O_4$ mixed metal oxides/ solid solution/ spinel structured oxides helps to decrease the availability of free hexavalent chromium (Cr6+) species (monomeric $CrO_4^{2-}$) in the catalyst and thereby minimizes the Cr leaching.
- Chromium leaching decreased by 1.5 times (pretreatment with air or nitrogen) and 16.2 times (pretreatment with hydrogen) by using structural modulator/ chromium stabilizing agent against the commercial catalyst composition.
- Rare-earth metal stabilized magnesium containing mesoporous microsphere alumina support provides improved hydrothermal stability and coke inhibiting properties in dehydrogenation catalysts.
- Incorporation of magnesium in the said support by using hydroxides or hydroxycarbonates of magnesium-aluminum as precursor offers better dispersion of Mg in the form of $MgAl_2O_4$ after calcination.
- High surface area of the said catalyst support enables better dispersion of active metal oxide sites to offer enhance catalytic performance.
- Nanocrystalline composite metal oxides by integration of $Cr_2O_3$ and $MCr_2O_4$ (M = Fe, Co, Ni, Cu and Zn) on the said catalyst support provided a unique catalytic behavior for propylene production from propane dehydrogenation at high propane concentration as feed (35-90%).
- The mixed metal oxide chemistry in catalyst preparation exhibited structural phase transformation from $Cr_2O_3$ to $MCr_2O_4$ by substitution of "Cr" with "M".
- The synergetic effect of "M" and "Cr" species in dehydrogenation catalyst facilitated better catalytic environment offering high olefin selectivity.
- The catalyst provides dual functionality namely mild acidity and dehydrogenation property to achieve high propane conversion and propylene selectivity.
- "M" species as structural modulator restricts the chromium leaching and improves the catalyst stability.
- Magnesium in the form of $MgAl_2O_4$ acts as a promoter to inhibit coke formation during reaction.
- The dehydrogenation catalyst in the present invention contains lower amount of chromium oxide content (≤ 12 wt.%) against the commercial catalyst containing 1-2% $K_2O/Na_2O$ and 18-20% $CrO_x$ (~23-26 wt.%$Cr_2O_3$) in the composition.
- The formation of $MCr_2O_4$ mixed metal oxides/ solid solution/ spinel structured oxides helps to reduce the availability of free hexavalent chromium (Cr6+) species in the catalyst and thereby minimize leaching of chromate species.
- Novel methodology to tune the hexavalent chromium (Cr6+) species in the catalyst composition was established with or without organic modulators like citric acid, maleic acid, ascorbic acid and more.

- Chromium leaching reduced by 1.5 times (pretreatment with air or nitrogen) and 16.2 times (pretreatment with hydrogen) by using "M" as stabilizing agent in the catalyst composition.
- Pretreatment of dehydrogenation catalysts prior to reaction with hydrogen showed less chromium leaching and better olefin selectivity compared to that of the fresh catalyst.

## EXAMPLES:

Example 1: Preparation of support-A $(Al_2O_3$, Mesoporous microsphere catalyst support) Step 1: Preparation of high surface area alumina precursor

In first step, 1000 gm of aluminum nitrate nonahydrate was dissolved in 7500 ml of deionized water and denoted as solution A. 10% soda lye solution or liquid ammonia and Solution A was added slowly into a vessel containing 1000 ml of deionized water by maintaining a pH of 9 - 11 at 30 °C and stirred for 3 - 5 hours and the reaction mixture was subjected to hydrothermal crystallization at 80 - 120 °C in an autoclave reactor for 1 - 24 hours. After cooling down the autoclave to room temperature, the crystallized product was withdrawn, filtered, and washed with hot deionized water repeatedly to eliminate unreacted reaction mixture. Then, the obtained cake was dried in an oven at 100 °C and crushed into fine powders.

Step 2: Preparation of $Al_2O_3$ microsphere catalyst support

650 - 850 grams, preferably 720 grams of dispersible alumina and 450 - 650 grams, preferably 550 grams of high surface area alumina precursor were added into a vessel containing 1900 ml of deionized water and stirred thoroughly; then the slurry was transferred to an attritor, and ball milled for 60 minutes. Then, 1050 ml of solution containing 50wt.% of aluminum nitrate was added into the milled slurry to initiate peptization process and continued stirring for 60 minutes to obtain homogenized binder and active alumina component. Then, the slurry with 19-22% solid content was spray dried and calcined at 500 - 900 °C, preferably 650 °C for 4 - 10 hours to obtain mesoporous microsphere catalyst support (20-150 μ).

## Example 2: Preparation of support-B (RE-$Al_2O_3$, Rare-earth metal stabilized mesoporous microsphere catalyst support)

Step 1: Preparation of 5wt.% RE (rare-earth elements, Ce or La) containing alumina precursor

In first step, 1000 gm of aluminum nitrate nonahydrate and the nitrates of rare-earth element referred to 5wt.% of rare-earth content was dissolved in 7500 ml of deionized water and denoted as solution A. 10% soda lye solution or liquid ammonia and Solution A was added slowly into a vessel containing 1000 ml of deionized water by maintaining a pH of 9 - 11 at 30 °C and stirred for 3 - 5 hours and the reaction mixture was subjected to hydrothermal crystallization at 80 - 120 °C in an autoclave reactor for 1 - 24 hours. After cooling down the autoclave to room temperature, the crystallized product was withdrawn, filtered, and washed with hot deionized water repeatedly to eliminate unreacted reaction mixture. Then, the obtained cake was dried in an oven at 100 °C and crushed into fine powders.

Step 2: Preparation of RE-$Al_2O_3$ microsphere catalyst support (RE = Ce or La)

650 - 850 grams, preferably 720 grams of dispersible alumina and 450 - 650 grams, preferably 550 grams of RE-containing alumina precursor were added into a vessel containing 1900 ml of deionized water and stirred thoroughly; then the slurry was transferred to an attritor, and ball milled for 60 minutes. Then, 1050 ml of solution containing 50 wt.% of aluminum nitrate was added into the milled slurry to initiate peptization process and continued stirring for 60 minutes to obtain homogenized binder and active alumina component. Then, the slurry with 19-22% solid content was spray dried and calcined at 500 - 900 °C, preferably at 650 °C for 4 - 10 hours to obtain mesoporous microsphere catalyst support (20-150 μ).

## Example 3: Preparation of Support-C (Mg-$Al_2O_3$, magnesium containing mesoporous microsphere catalyst support)

Step 1: Preparation of magnesium-aluminum hydroxycarbonates

In a typical synthesis, 60 to 400 g of magnesium nitrate hexahydrate and 192 g aluminum nitrate nonahydrate were dissolved in 1000 ml of deionized water (denoted as Solution-A2). Solution-B2 was prepared by dissolving 40 to 120 g of sodium hydrogen carbonate in 1000 ml deionised water. Then, solution A2 and solution B2 were simultaneously added dropwise into a reaction vessel containing 500 ml of deionized water under stirring at 35 °C. During co-precipitation, the pH of the slurry was maintained at 10 by adding 2 to 10 M NaOH (sodium hydroxide) aqueous solution under stirring. After precipitation, the resultant slurry was aged hydrothermally at 80 - 140 °C, preferably 100 °C for 24 hours. Later, after completion of reaction, the reactor was cooled to room temperature, then the crystallized material was filtered and washed with deionized water several times until the pH of the filtrate was neutral. The obtained filtered solid was dried in an oven at 100 °C and crushed into fine powders.

Step 2: Preparation of magnesium-aluminum hydroxide

60 to 400 g of magnesium nitrate hexahydrate and 192 g aluminum nitrate nonahydrate were dissolved in 1000 ml of deionized water (denoted as Solution-A3). Then, solution-A3 was added dropwise into a reaction vessel containing 500 ml of deionized water under stirring at 35 °C. During co-precipitation, the pH of the slurry was maintained at 10 by adding 2 to 10 M NaOH (sodium hydroxide) aqueous solution under stirring. After precipitation, the resultant slurry was aged hydrothermally at 80 - 140 °C, preferably 100 °C for 24 hours. Then, after cooling to room temperature, the crystallized material was filtered and washed with deionized water for several times until the pH of the filtrate was neutral. The obtained filtered solid was dried in an oven at 100 °C and crushed into fine powders.

Step 3: Preparation of magnesium containing mesoporous microsphere catalyst support, Mg-Al$_2$O$_3$) (Catalyst support-C)

500 - 700 grams, preferably 580 grams of dispersible alumina, 500 - 700 grams, preferably 550 grams of high surface area alumina precursor (as prepared in Step 1 of Example 1) and 100 - 200 grams, preferably 125 grams of magnesium-aluminum hydroxide or magnesium-aluminum hydroxycarbonates were mixed and stirred with 1900 ml of deionized water; then the slurry was transferred to an attritor, and ball milled for 60 minutes. Then, 1050 ml of solution containing 50wt.% of aluminum nitrate was added into the milled slurry to initiate peptization process and stirred for 60 minutes to obtain homogenized binder and active alumina component. Then, the slurry with 19 - 22% solid content was spray dried and calcined at 500 - 900 °C, preferably at 650 °C for 4 - 10 hours to obtain mesoporous microsphere catalyst support C (20-150 μ).

**Example 4: Preparation of support-D (RE-Mg-Al$_2$O$_3$, rare-earth metal stabilized magnesium containing mesoporous microsphere catalyst support)**

Step 1: Preparation of RE-Mg-Al$_2$O$_3$ microsphere catalyst support (RE = Ce or La)

500 - 700 grams, preferably 580 grams of dispersible alumina, 500 - 700 grams, preferably 550 grams of RE-containing alumina precursor (as prepared in Step 1 of Example 2) and 100 - 200 grams, preferably 125 grams of magnesium-aluminum hydroxide (as prepared in Step 1 of Example 3) or hydroxycarbonates (as prepared in Step 2 of Example 3) was mixed and stirred with 1900 ml of deionized water; then the slurry was transferred into an attritor, and ball milled for 60 minutes. Then, 1050 ml of solution containing 50 wt.% of aluminum nitrate was added into the milled slurry to initiate peptization process and continued stirring for 60 minutes to obtain homogenized binder and active alumina component. Then, the slurry with 19 - 22% solid content was spray dried and calcined at 500 - 900 °C, preferably at 650 °C for 4 - 10 hours to obtain rare-earth stabilized magnesium containing mesoporous microsphere catalyst support D (20-150 μ).

**Example 5: Preparation of Support-E (ZnO-RE-Mg-Al$_2$O$_3$, ZnO-modified rare-earth metal stabilized magnesium containing mesoporous microsphere catalyst)**

A transition metal nitrate (Fe, Co, Ni, Cu and Zn), preferably zinc nitrate hexahydrate corresponding to 2 - 4 wt.% ZnO was dissolved in deionized water and impregnated by spraying the metal solution on rare-earth metal stabilized magnesium containing mesoporous microsphere catalyst support (as prepared in Example 4, Support-D) with continuous mixing to achieve uniform distribution of metal species. Later, the impregnated wet catalyst was dried at 120 °C overnight and calcined at 500 - 900 °C, preferably at 650 °C for 4 - 10 hours.

| Table 1. Physico-chemical properties of catalyst support | | | | | | | | | |
|---|---|---|---|---|---|---|---|---|---|
| Example | Support | Description | SA (m$^2$/g) | PV (cc/g) | Barrett-Joyner-Halenda (BJH) distribution (%) | | | | Acidity ($\mu$mol NH3 desorbed/g) |
| | | | | | <50 Å | 50-100 Å | 100-180 Å | >180 Å | |
| Example 1 | Support-A | Al$_2$O$_3$ | 155 | 0.34 | 25.4 | 59.6 | 12.4 | 2.6 | 400 |
| Example 2 | Support B | RE-Al$_2$O$_3$ | 168 | 0.38 | 29.5 | 54.2 | 13 | 3.3 | 380 |
| Example 3 | Support-C | Mg-Al$_2$O$_3$ | 172 | 0.39 | 26.5 | 54.4 | 13.8 | 5.3 | 300 |
| Example 4 | Support-D | RE-Mg-Al$_2$O$_3$ | 181 | 0.42 | 25.8 | 58.6 | 12.7 | 2.9 | 320 |
| Example 5 | Support-E | ZnO-RE-Mg-Al$_2$O$_3$ | 150 | 0.34 | 23.4 | 54.2 | 17.3 | 5.1 | 325 |

The physico-chemical properties of the catalyst support(s) were characterized using nitrogen sorption techniques (surface area - SA, pore volume - PV and pore size distribution) and ammonia-temperature programmed desorption technique (NH$_3$-TPD); and the results are tabulated in Table 1. The results show that the catalyst support exhibits mesoporosity with the pore volume ranging from 0.34 - 0.42 cc/g and surface area ranging from 150 - 181 m$^2$/g. The acidity of the catalyst support was ranging from 300 - 400 $\mu$mol NH$_3$ desorbed/g. Based on the higher surface area (172 - 181 m$^2$/g) and relatively moderate amount of acidity (300 - 320 $\mu$mol/g), the Support-C and Support-D namely Mg-Al$_2$O$_3$ and RE-Mg-Al$_2$O$_3$ are selected for further experiments.

**Example 6: Preparation of alkane dehydrogenation catalysts**

CAT-A: Preparation of Cr$_2$O$_3$-MO supported on Mg-Al$_2$O$_3$ (Support-C)

100 gm of calcined support-C (prepared under Example 3) was used for catalyst preparation. The chromium nitrate nonahydrate corresponding to 8 - 12 wt.% Cr$_2$O$_3$ and either of other transition metal nitrates namely Fe, Co, Ni, Cu and Zn, corresponding to 2 - 4 wt.% of metal oxide (MO) was dissolved in deionized water referred to the pore volume of catalyst support. Then, the prepared mixed metal nitrate solution was impregnated by spraying the solution on catalyst support with continuous mixing to achieve uniform distribution of active catalytic components. Later, the obtained impregnated wet catalyst was dried at 120 °C overnight and calcined at 500 - 900 °C, at preferably 650 °C for 2 - 10 hours.

CAT-B: Preparation of Cr$_2$O$_3$ supported on RE-Mg-Al$_2$O$_3$ (Support-D)

100 gm of support-D (prepared under Example 4) was used for catalyst preparation. The chromium nitrate nonahydrate corresponding to 8 - 12 wt.% Cr$_2$O$_3$ was dissolved in deionized water referred to pore volume of catalyst support. Then, the chromium nitrate solution was impregnated by spraying the solution on the catalyst support with continuous mixing to achieve uniform distribution of active catalytic components. Later, the impregnated wet catalyst was dried at 120 °C overnight and calcined at 500 - 900 °C, preferably at 650 °C for 2 - 10 hours.

CAT-C (CAT-C1 to CAT-C5): Preparation of Cr$_2$O$_3$-MO supported on RE-Mg-Al$_2$O$_3$ (Support-D)

100 gm of calcined support-D (prepared under Example 4) was used for catalyst preparation. The chromium nitrate nonahydrate corresponding to 8 - 12 wt.% Cr$_2$O$_3$ and either of other transition metal nitrates namely Fe (described as CAT-C1), Co (described as CAT-C2), Ni (described as CAT-C3), Cu (described as CAT-C4) and Zn (described as CAT-C5), corresponding to 2 - 4 wt.% of metal oxide (MO) was dissolved in deionized water referred to the pore volume of catalyst support. Then, the prepared mixed metal nitrate solution was impregnated by spraying the solution on catalyst support with continuous mixing to achieve uniform distribution of active catalytic components. Later, the obtained impregnated wet catalyst was dried at 120 °C overnight and calcined at 500 - 900 °C, preferably at 650 °C for 2 - 10 hours. CAT-D: Preparation of Cr$_2$O$_3$-MO supported on RE-Mg-Al$_2$O$_3$ (Support-D) by metal chelate complex.

100 gm of calcined support-D (prepared under Example 4) was used for catalyst preparation. Solution A4 containing organic acid preferably 10 - 20 grams of citric acid monohydrate was dissolved in deionized water referred to water uptake of catalyst support. Then, the chromium nitrate nonahydrate (corresponding to 8 - 12wt.% $Cr_2O_3$) and either of other transition metal nitrates namely, Fe, Co, Ni, Cu and Zn, preferably Zn nitrate (corresponding to 2 - 4 wt.% of support) added into solution A4 and mixed thoroughly to dissolve completely to form metal-chelate complex. Then, the prepared metal-chelate complex containing mixed metals solution was impregnated by spraying the solution on the catalyst support with continuous mixing to achieve uniform distribution. Later, the obtained

impregnated wet catalyst was dried at 120 °C overnight and calcined at 500 - 900 °C, preferably at 650 °C for 2 - 10 hours.

CAT-E: Preparation of 2% $K_2O$/ $Na_2O$ - 20 wt.% $Cr_2O_3$ supported $Al_2O_3$ support.

100 gm of calcined support-A (prepared under Example 1) was used for catalyst preparation. The required quantity of chromium nitrate nonahydrate and potassium or sodium nitrate was dissolved in the required volume of deionized water referred to pore volume of catalyst support. Then, the metal nitrate solution was impregnated by spraying the solution on the catalyst support with continuous mixing to achieve uniform distribution. Later, the impregnated wet catalyst was dried at 120 °C overnight and calcined at 500 - 900 °C, preferably at 650 °C for 2 - 10 hours.

**Example 7: Determination of leachable hexavalent chromium Cr(6+) species in dehydrogenation catalysts by metal extraction method.**

This example illustrates the chromium stability in dehydrogenation catalysts which are prepared under Example 6. The leachable hexavalent chromium Cr(6+) in dehydrogenation catalyst was extracted with deionized water. Prior to the experiment, the catalyst was pretreated either under air or nitrogen at a temperature about 600 - 700 °C, preferably at 650 °C for 45 minutes or reduced under hydrogen gas at a temperature about 600 - 700 °C, preferably at 650 °C for 45 minutes and then purged with nitrogen for 30 minutes. In a typical Cr(6+) extraction experiment, 10 grams of pretreated catalyst was added into a beaker containing 50 ml of deionized water and stirred using a glass rod. Then allowed to dissolve the Cr(6+) species in deionized water for 30 minutes and allowed to settle down the catalyst particles. Later, the supernatant solution was collected and repeated the procedure until a clear solution was observed.

Example/Serial 7 of Table 2 shows the percentage (%) of Cr(6+) species leached referred to the total chromium content in dehydrogenation catalysts. Notably, the pretreatment of dehydrogenation catalysts using air or nitrogen gas showed Cr(6+) leaching ranging from 10.5 to 14.1% based on the type of transition metals as structural modulators and different catalyst supports employed during the catalyst preparation. Most importantly, the dehydrogenation catalyst (CAT-E) containing commercial catalyst composition of 2%$K_2O$/$Na_2O$-20%$Cr_2O_3$ supported on alumina showed Cr(6+) leaching of 17.0% with pre-treatment using air or nitrogen.

In another experiment, the dehydrogenation catalysts were subjected to reduction by pre-treating the catalysts using hydrogen gas to convert the hexavalent chromate species namely $CrO_4^{2-}$ (Cr6+) into trivalent chromium oxide $Cr_2O_3$ (Cr3+). As a result, a significant improvement in chromium stability was observed with the dehydrogenation catalysts using transition metals as structural modulators, wherein the said catalysts showed a negligible Cr(6+) leaching ranging from 0.5 to 1.8% against the commercial dehydrogenation catalyst composition showing Cr(6+) leaching around 8.1%. This indicates the existence of higher amount of Cr(6+) namely $CrO_4^{2-}$ species in the commercial catalyst composition even after pretreatment with hydrogen. In overall, the pretreatment of dehydrogenation catalysts using hydrogen gas converts the undesirable Cr(6+) [($CrO_4^{2-}$) (Cr6+) into $Cr_2O_3$ (Cr3+)] compared to the catalysts pretreated either with air or nitrogen, indicating the influence of pretreatment conditions in catalyst composition.

**Table 2: Results on Chromium (6+) leaching studies of dehydrogenation catalysts**

| S. No. | CATALYST | Active metal oxide composition | | Support | Chromium (6+) leached (wt.%) # | |
|---|---|---|---|---|---|---|
| | | $Cr_2O_3$ | MO | | Pretreatment with air or nitrogen * | Pretreatment with hydrogen * |
| 1 | CAT-A | $Cr_2O_3$ | ZnO | $Mg-Al_2O_3$ | 11.0 | 0.9 |
| 2 | CAT-B | $Cr_2O_3$ | - | $RE-Mg-Al_2O_3$ | 10.5 | 0.8 |
| 3 | CAT-C 1 | $Cr_2O_3$ | FeO | $RE-Mg-Al_2O_3$ | 12.2 | 0.8 |
| 4 | CAT-C2 | $Cr_2O_3$ | CoO | $RE-Mg-Al_2O_3$ | 12.7 | 1.4 |
| 5 | CAT-C3 | $Cr_2O_3$ | NiO | $RE-Mg-Al_2O_3$ | 14.1 | 1.8 |
| 6 | CAT-C4 | $Cr_2O_3$ | CuO | $RE-Mg-Al_2O_3$ | 13.6 | 0.7 |
| 7 | CAT-C5 | $Cr_2O_3$ | ZnO | $RE-Mg-Al_2O_3$ | 10.6 | 0.5 |
| 8 | CAT-D | $Cr_2O_3$ | ZnO-CA | $RE-Mg-Al_2O_3$ | 11.2 | 0.8 |
| 9 | CAT-E | $Cr_2O_3$ | $K_2O/ Na_2O$ | $Al_2O_3$ | 17.0 | 8.1 |

$$\text{\# } \textbf{\textit{Chromium (Cr6+) leached (\%)}} = \frac{Amount\ of\ Chromium\ (Cr6+)\ leached}{Total\ Chromium\ in\ catalyst\ composition} X\ \textbf{100}$$

*Pretreatment at temperature about 650 °C for 45 minutes

**Example 8: Hydrothermal stability test of dehydrogenation catalyst**

The hydrothermal stability of selected dehydrogenation catalysts was carried out in the auto-steaming unit. In a typical experiment, 25 grams of catalyst was loaded into a tubular reactor and heated at a temperature of about 700 - 800 °C under the flow of nitrogen gas. After reaching the temperature of about 700 - 800 °C, the catalyst was reduced using hydrogen gas for 1 hour at 700 - 800 °C. Later, hydrogen flow was stopped and then steam was introduced into the reactor at a rate of 4 ml/hour for 2 - 4 hours at a temperature of about 700 - 800 °C. Then the reactor was cooled down to room temperature and the hydrothermally treated catalyst was characterized and the results are tabulated in Table 3.

The hydrothermal stability of selected dehydrogenation catalysts prepared by employing with and without rare-earth metal stabilized magnesium containing alumina support showed an interesting characteristic property as mentioned in Table 3. The rare-earth metal stabilized catalyst CAT-C5 retained its porous properties with a loss of ~10% surface area against the fresh catalyst, however the non-stabilized catalyst CAT-A showed a loss of 26%. Notably, CAT-E showed ~21% loss in surface area after hydrothermal treatment. The studies shows that the CAT-5 has higher hydrothermal stability compared to that of other catalysts.

| Table 3: Physico-chemical properties of selected dehydrogenation catalysts | | | |
|---|---|---|---|
| **Description** | **CAT-A** | **CAT-C5** | **CAT-E** |
| Active component | $Cr_2O_3$-ZnO | $Cr_2O_3$-ZnO | $Cr_2O_3/K_2O$ |
| Support | Mg-$Al_2O_3$ | RE-Mg-$Al_2O_3$ | $Al_2O_3$ |
| $S_{BET}$ ($m^2$/g) Fresh | 135 | 130 | 115 |
| $S_{BET}$ ($m^2$/g) Steamed* | 100* | 118* | 90* |
| Pore volume (cc/g) | 0.29 | 0.31 | 0.27 |
| Pore volume (cc/g) Steamed* | 0.26* | 0.30* | 0.26* |
| ABD (g/cc) ($\mu$m) | 1.00 | 1.00 | 0.95 |
| Average particle size (APS) | 78 | 78 | 74 |
| Attrition Index (ASTM D5757) ($\mu$m) | 2.0 | 1.5 | 1.5 |
| TPR ($\mu$mol/g) | 300 | 320 | 380 |
| Acidity ($\mu$mol/g) | 375 | 310 | 300 |
| $(C_r^{6+})$ leached referred to total Cr content in catalyst | 11.0 | 10.6 | 17.0 |
| with pretreatment using air or nitrogen (%) | | | |
| $(C_r^{6+})$ leached referred to total Cr content in catalyst after pre-treatment using hydrogen[#] (%) | 0.9%[#] | 0.5%[#] | 8.1%[#] |
| *Hydrothermal stability test (Reduced at 700 °C for 1 hour & steamed at 700 °C for 2 hours) [#]Reduced using hydrogen at 650 °C for 45 minutes. | | | |

**Example 9: XRD analysis of dehydrogenation catalyst support and catalysts**

This example illustrates the crystallographic phase identification of dehydrogenation catalysts, and its catalyst support investigated by X-ray diffraction (XRD) measurements and depicted in Fig. 1. of Example 9. The XRD patterns of dehydrogenation catalyst supports namely Support-C (Mg/$Al_2O_3$) and Support-D (RE-Mg-$Al_2O_3$) showed the diffraction peaks corresponding to gamma-alumina phase. Notably, CAT-B (8-12 wt.% $Cr_2O_3$/Ce-Mg-$Al_2O_3$) showed the absence of diffraction peaks corresponding to $Cr_2O_3$ despite loading of 8 - 12wt.%$Cr_2O_3$ on catalyst support, suggesting the fine dispersion of $Cr_2O_3$ on mesoporous support leading to amorphous nature. The dehydrogenation catalyst of CAT-A and CAT-C containing 8 - 12wt.% $Cr_2O_3$ and 2 - 4wt.% ZnO showed the peaks corresponding to cubic phase of $ZnCr_2O_4$ mixed metal oxide and gamma-alumina. The spinel structured zinc chromium oxide ($ZnCr_2O_4$) formation is attributed to the isomorphous substitution of Zn(2+) ions in $Cr_2O_3$ lattice (Cr3+ sites) leads to the phase transition from $Cr_2O_3$ to $ZnCr_2O_4$. This suggests that the phase transition of crystal structure occurs from $Cr_2O_3$ to $ZnCr_2O_4$ solid solutions or mixed metal oxide upon addition of other transition metal precursor along with chromium precursor during the catalyst preparation. More importantly, the peaks corresponding to the remaining amount of unreacted $Cr_2O_3$ were not detected in XRD measurements since the ZnO-to-$Cr_2O_3$ ratio was varied from 1 (ZnO)-to-3 ($Cr_2O_3$) to 4 (ZnO)-to-3 ($Cr_2O_3$) ratio during the

EP 4 786 045 A2

catalyst preparation; this suggests that the catalyst also contains amorphous nature of $Cr_2O_3$ or other metal oxides on mesoporous catalyst support. Notably, the XRD analysis indicates that the catalyst system consists of composite metal oxide, which is composed of nanocrystalline $ZnCr_2O_4$, amorphous $Cr_2O_3$ and nanocrystalline $Al_2O_3$.

**Example 10: Performance evaluation of alkane dehydrogenation catalysts**

The catalysts of present invention are effective as dehydrogenation catalysts and are effective in promoting the dehydrogenation of alkanes namely ethane, propane, iso-butane, n-butane and iso-pentane to produce the respective olefins or diolefins. The performance of catalyst was evaluated in a fixed bed or fluidized reactor system at simulated process conditions. Prior to the dehydrogenation reaction, the catalyst was pre-treated either under hydrogen or without hydrogen gas at 600 - 750° C. Then, the catalyst was evaluated at reaction temperature ranging from 580 to 650° C using a feed stream containing 35-90% of alkane balanced in nitrogen gas at gas hourly space velocity (GHSV) of the feed gas of 2000 - 5000 $h^{-1}$.

Example 10 shows the efficacy of dehydrogenation catalysts and effect of pretreatment conditions on dehydrogenation catalysts with and without reducing gas on the catalytic performance. The evaluation was performed at the process conditions at 20 minutes reaction time and the results are shown in Table 4.

13

Table 4. Performance testing results of dehydrogenation catalysts

| Catalysts | Active metal oxide composition | Support | Acidity ($\mu$mol/g) | ABD (g/cc) | Catalyst pretreatment without hydrogen | | | Catalyst pretreatment with hydrogen | | | Coke (wt%) |
|---|---|---|---|---|---|---|---|---|---|---|---|
| | | | | | Propane conv. (%) | Propylene (%) | | Propane conv. (%) | Propylene (%) | | |
| | | | | | | Sel. | Yield | | Sel. | Yield | |
| CAT-B | $Cr_2O_3$ | $RE\text{-}Mg\text{-}Al_2O_3$ | 375 | 1.00 | 49.5 | 73.0 | 36.1 | 39.6 | 82.5 | 32.7 | 2.5 |
| CAT-C5 | $ZnO\text{-}Cr_2O_3$ | $RE\text{-}Mg\text{-}Al_2O_3$ | 310 | 0.98 | 55.0 | 80.5 | 44.3 | 46.0 | 88.2 | 40.6 | 1.2 |
| CAT-E | $K_2O/\ Na_2O\text{-}20\%Cr_2O_3\text{-}$ | $Al_2O_3$ | 300 | 0.94 | 53.1 | 78.8 | 41.8 | 43.8 | 89.0 | 39.0 | 0.7 |

As shown in Table 4, the dehydrogenation catalysts namely CAT-B and CAT-C5 prepared using rare-earth metal stabilized magnesium containing alumina support (Support-C) showed a propane conversion from 49 to 55% and propylene selectivity from 73 to 80% using catalyst without hydrogen pre-treatment. Notably, the CAT-C5 containing Zn as a structural modulator for $Cr_2O_3$ in the catalyst formulation showed a relative improvement in propane conversion and propylene selectivity compared to CAT-B containing $Cr_2O_3$ as a sole active dehydrogenating component. Further, the pretreatment of dehydrogenation catalysts with hydrogen gas plays a vital role in catalytic activity in addition to the catalyst composition. CAT-C5 showed a remarkable improvement in propylene selectivity of 88% upon pretreatment of catalyst with hydrogen gas, whereas the catalysts without hydrogen gas pretreatment exhibited 80% propylene selectivity.

## Claims

1. A composite catalyst for non-oxidative dehydrogenation of lighter alkanes, comprising:

   a) a catalyst support in an amount of 70-90wt.% of the composite catalyst, wherein the catalyst support comprises one or more rare-earth metal in an amount of 0.5-10 wt% of the composite catalyst, and one or more alkaline-earth metal in an amount of 0.5-10 wt% of the composite catalyst;
   b) a first transition metal oxide in an amount of 5-25 wt.% of the composite catalyst; and
   c) one or more second transition metal oxide in an amount of 1-10 wt.% of the composite catalyst.

2. The composite catalyst as claimed in claim 1, wherein the catalyst support is selected from alumina or aluminum oxide, aluminum monohydrate, aluminum trihydrate, gamma-alumina, eta-alumina, delta-alumina, silica-alumina and zeolites; wherein the rare-earth metals are selected from cerium, lanthanum, yttrium, scandium, samarium or mixtures thereof; and wherein the alkaline earth metals are selected from magnesium, calcium, strontium, rubidium, barium, or mixtures thereof.

3. The composite catalyst as claimed in claim 1, wherein the first transition metal oxide is in the range of 8-12 wt.% of the composite catalyst and is chromium oxide; and wherein the second transition metal oxide is in the range of 2-4 wt.% of the composite catalyst and is selected from iron oxide, cobalt oxide, nickel oxide, copper oxide, zinc oxide or mixtures thereof.

4. A process for preparing a composite catalyst for non-oxidative dehydrogenation of lighter alkanes, comprising the steps of:

   i. dissolving 42.1-63.2 grams of chromium nitrate nonahydrate and 7.3-14.7 grams of at least one second transition metal nitrate in deionized water to obtain a mixed metal nitrate solution;
   ii. impregnating the mixed metal nitrate solution by spraying on a calcined catalyst support-D to obtain an impregnated catalyst support-D;
   iii. drying the impregnated catalyst support-D at 100-150 °C overnight to obtain a dried catalyst, and
   iv. calcining the dried catalyst at a temperature in the range of 500 - 900 °C for 2 - 10 hours to obtain the composite catalyst having particle size about 20 to 150 $\mu$m.

5. The process as claimed in claim 4, wherein the process used a fixed bed or fluidization reactor system.

6. The process as claimed in claim 4, wherein the chromium nitrate nonahydrate corresponds to 8-12 wt.% $Cr_2O_3$ in the total composite catalyst; and wherein the second transition metal nitrates correspond to 2-4 wt.% of transition metal oxide (MO) in the total composite catalyst; wherein transition metal in the transition metal nitrate and in the transition metal oxide is selected from iron, cobalt, nickel, copper and zinc.

7. The process as claimed in claim 4, wherein the impregnated catalyst support-D is dried at a temperature of 120 °C; and wherein the calcination is performed at a temperature of 650 °C for 2-10 hours.

8. The process as claimed in claim 4, wherein the calcined catalyst support-D is prepared by a process comprising the steps of:

   i. mixing 500 - 600 grams of dispersible alumina, 500 - 600 grams of rare-earth (RE)-containing alumina precursor, 100 - 200 grams of magnesium-aluminum hydroxide or hydroxycarbonates, and 1800 - 2000 ml of deionized water to obtain a slurry;

ii. transferring the slurry into an attritor and ball milling the slurry for 50 - 70 minutes to obtain a milled slurry;

iii. adding 1000 - 1100 ml of a solution containing 40-60 wt.% of aluminum nitrate to the milled slurry with continuous stirring for 50-80 minutes to obtain a homogenized slurry;

iv. spray drying the slurry followed by calcining at a temperature in the range of 500 - 900 °C for 4 - 10 hours to obtain the calcined catalyst support D.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040242945 A1
- US 20130165729 A1
- US 11040338 B2
- US 4041099 A
- CN 111408370 A
- US 20100010280 A1
- US 8063261 B2
- US 8101541 B2
- US 20050075243 A1
- US 20200147588 A1
- US 20210001316 A
- US 20100312035 A1
- US 20030232720 A1
- US 8895468 B2
- WO 2014046659 A1